Europäisches Patentamt

European Patent Office

Office européen des brevets

(19)

(11) Veröffentlichungsnummer: **0 127 102 B1**

(12) **EUROPÄISCHE PATENTSCHRIFT**

(45) Veröffentlichungstag der Patentschrift: 02.05.90

(21) Anmeldenummer: **84105787.0**

(22) Anmeldetag: **21.05.84**

(51) Int. Cl.⁵: **A 61 F 13/04**, A 61 F 13/00, D 04 B 1/04, D 04 B 21/04

(54) **Medizinischer Polsterschlauch und Verfahren zu dessen Herstellung.**

(30) Priorität: **27.05.83 CH 2929/83**

(43) Veröffentlichungstag der Anmeldung:
**05.12.84 Patentblatt 84/49**

(45) Bekanntmachung des Hinweises auf die
Patenterteilung:
**02.05.90 Patentblatt 90/18**

(84) Benannte Vertragsstaaten:
**AT BE DE FR GB IT LU NL SE**

(56) Entgegenhaltungen:
EP-A-0 086 960
CH-A- 612 345
DE-A-2 059 061
DE-A-2 722 336
DE-B-1 165 201
GB-A- 840 523
GB-A-1 557 328
GB-A-2 113 976
US-A-3 724 457
US-A-4 150 442
US-A-4 253 317

(73) Patentinhaber: **Baumann AG, Verbandstoffabrik
Hofackerstrasse 44
CH-8032 Zürich (CH)**

(72) Erfinder: **Baumann, Rolf E.
Heliosstrasse 10
CH-Zürich (CH)**

(74) Vertreter: **Monsch, René et al
E. BLUM & CO., Patentanwälte Vorderberg 11
CH-8044 Zürich (CH)**

Courier Press, Leamington Spa, England.

## Beschreibung

Die Erfindung betrifft einen medizinischen Polsterschlauch aus gewirktem bzw. gestricktem Textilstoff, der nahtlos zum Schlauch geschlossen ist.

In der Medizin sind verschiedene schlauchartige Textilgebilde bekannt, die als Bandagen oder als Zwischenlagen bei Gipsverbänden verwendet werden. Bei bekannten Bandagen dieser Art wird regelmässig eine Kompression angestrebt, wie sich beispielsweise aus der CH—PS 612 345 ergibt. Die als Zwischenlagen bei Gipsverbänden verwendeten Gazeschläuche weisen zwar keinen solchen Kompressionseffekt auf, erfordern jedoch zusätzlich lokale Polsterungen.

Es sind bereits verschiedene, mehr oder weniger schlauchförmige Textilgebilde in verschiedenen weiteren Ausgestaltungen bekannt geworden, wie etwa als Schweissbänder (DE—A 2 722 336), Wundverbände (DE—A 2 059 061), Zirkulärbandagen (CH—A 612 345, GB—A 840 523) zum Fixieren von Verbänden (US 3 724 457) oder als Ellbogen- bzw. Knieschoner (US—PS 4 150 442). Einige dieser Gebilde DE—A 2 722 336, DE—A 2 059 061, CH—A 612 345, GB—A 840 523) weisen ihrem Anwendungszweck entsprechend eine relativ starke, radiale Kompressionswirkung auf, die durch umlaufende, elastische Fäden verursacht wird, die nicht in den Maschenreihen verlaufen, sondern sich quer durch diese hindurch erstrecken und neben dem Gewirke eine zweite Struktur von zueinander parallel verlaufenden, untereinander nicht verbundenen Fäden bilden. Damit wird praktisch keine Elastizität in Schlauchlängsrichtung und eine starke radiale Kompression bewirkt.

In anderen dieser Gebilde sind nur einzelne Teilbereich mit elastischen Fäden versehen, die zwar untereinander verwirkt sind, jedoch in einer Weise, welche eine relativ kleine Längselastizität nach sich zieht (US—A 4 150 442, US—A 4 253 317).

Es stellt sich die Aufgabe, einen medizinischen Polsterschlauch zu schaffen, der einerseits keine unerwünschten Kompressionswirkungen hat, sich jedoch zugleich faltenlos an die gewünschten Körperpartien anlegt, um beispielsweise als Polster für einen Gipsverband oder als Grundlage für bestimmte Verbände, wie z.B. den Gilchrist-Verband, zu dienen.

Diese Aufgabe wird beim eingangs erwähnten Polsterschlauch dadurch gelöst, dass jede der umlaufenden Maschenreihen von mindestens einem elastischem Element und mindestens einem Strickfaden gebildet ist, wobei die Maschen Rechts-Links-Bindung aufweisen und als elastisches Element ein Coregarn mit baumwollumsponnenem, elastischen Kern vorgesehen ist und wobei der Strickfaden auf mindestens einer Seite des Textilstoffes eine frottéartige Struktur bildet, derart, dass der Schlauch längs- und querdrehbar ist, wobei das elastische Dehnungsverhältnis in Längsrichtung grösser ust als in Querrichtung.

Vorzugsweise ist das elastische Dehnungsverhältnis des Schlauches, d.h. der Bruchteil elastischer Dehnung an der Gesamtdehnung in der Querrichtung relativ klein, so dass sich der Schlauch zwar den Körperformen gut anpasst, jedoch trotzdem keine unerwünschte Kompression erzeugt.

Als elastisches Element wird vorzugsweise eine umsponnene Elastomerfaser aus Polyurethan verwendet, wie sie unter der Bezeichnung Lycra erhältlich ist.

Der Strickfaden besteht bei bevorzugten Ausführungen aus Baumwolle. Für eine etwas schwerere Qualität mit erhöhtem Polstereffekt können in jeder Maschenreihe zwei Strickfäden vorgesehen sein.

Die Herstellung des Polsterschlauches kann auf einer Rundstrickmaschine in gefachter Verarbeitungsart erfolgen.

Nachfolgend werden Ausführungsbeispiele der Erfindung beschrieben, wobei zunächst ein Polsterschlauch für die Extremitäten erläutert wird, der zugleich für Arm und Beinpartien geeignet ist.

Der Schlauch weist ungedehnt eine Breite von ca. 8 cm auf, sowie eine an sich beliebige Länge. Seine textilen Eigenschaften ergeben sich aus der nachfolgenden tabellarischen Uebersicht: (PU=Polyurethan).

| | |
|---|---|
| Breite ungedehnt | 8,3 cm |
| Breite gedehnt | 30,7 cm |
| Längsdehnbarkeit | 180% |
| Querdehnbarkeit | 270% |
| m²-Gewicht ungedehnt | 1140 g |
| m²-Gewicht gedehnt | 110 g |
| Maschenreihen/10 cm unged. | 84 |
| Maschenreihen/10 cm ged. | 33 |
| Maschenstäbe/Umfang | 112 |
| Garnstärke Textil | 30 tex ×1, Baumwolle, T/m z 820 |
| Kulierverbrauch/ Umfang | Textil—1,43 m Coregarn—0,166 m |
| Kulierverbrauch/M | Textil—1201,2 m Coregarn—139,44 m |

| | |
|---|---|
| Elastisches element: | |
| Tex PU nackt un- gedehnt | 5,9 |
| Tex PU nackt gedehnt | 2,0 |
| Verzug | 1:3,0 |
| Tex Umspinnung | 24,8 tex, Baumwolle |
| Endtex gedehnt | 26,8 |
| Verarbeitungsart | gefacht |
| Systeme | (+1 elastisches Element) |
| Farbe | rohweiss |
| Maschine | Rundstrickmaschine |
| Bindung | rechts/links |
| Dicke einfach | 2,29 mm |

%-Anteile:

| | |
|---|---|
| Textil (Baumwolle) | 78% |
| "Umspinnung" (Baumwolle) | 20% |
| Polyurethan | 2% |

Bemerkung:

Das Coregarn liegt mit 144 Endtex ungedehnt in der Ware—in jeder Maschenreihe ein elastisches Element eingelegt.

Im Ausgangszustand ist das elastische Element nicht gedehnt.

Wie sich aus dieser Tabelle ergibt, ist eine grosse Querdehnbarkeit vorhanden, welche eine optimale Anpassung an verschiedene Gliederpartien und Gliederstärken erlaubt, so dass eine einzige Schlauchgrösse dafür ausreicht. Die Querdrehnung erfolgt dabei nicht vollständig elastisch, sondern es verbleibt bei Querdehnung eine sich nicht elastisch zurückbildende Restdehnung, was bei der Anwendung sicherstellt, dass keine unerwünschte Kompression auftritt. Die Restdehnung kann durch Streckung in Längsrichtung weitgehend beseitigt werden. Der verwendete Textilstoff ist hautverträglich und durchlässig für Röntgenstrahlung, so dass sich insbesondere bei Verwendung als Polsterung bei Gipsverbänden keine Behinderung der Röntgendiagnose ergibt. Der Polster-Effekt ergibt sich durch die frottéeartige Oberflächenstruktur auf der Innenseite des Schlauches, die zu einer Stoffdicke von ca. 2,3 mm führt. Diese frottéeartige Struktur wird durch die im ungedehnten Zustand des elastischen Elementes nach innen herausragende Maschenköpfe des Strickfadens gebildet. Die Schlauchaussenseite besitzt dagen eine relativ glatte Struktur, so dass die Gleitfähigkeit auf der Aussenseite gut bleibt. Bei der Herstellung von Gipsverbänden kann damit eine separate lokale Polsterung wegfallen, wodurch das Vorgehen wesentlich vereinfacht wird. Ein auf die entsprechende Länge zugeschnittener Schlauchteil wird dazu über die Extremität gezogen und an der gewünschten Stelle plaziert. Der Polsterschlauch kann dann mit Krepp-Papier umwickelt werden, wonach der Gipsverband angebracht wird.

Der beschriebene Polsterschlauch ist allgemein dort anwendbar, wo Extremitäten ohne wesentliche Kompressionswirkung schlauchartig umfasst werden sollen, ohne dass sich dabei Falten und Druckstellen ergeben. Dies ist insbesondere auch bei bestimmten Verbänden, wie dem sogenannten Gilchrist-Verband zur Ruhigstellung des Arms, von Bedeutung, wo der Arm von Teilen des Verbandes schlauchartig umfasst und von anderen Teilen in angewinkelter Lage gehalten wird.

Während beim beschriebenen Ausführungsbeispiel pro Maschenreihe ein Strickfaden und ein elastisches Element vorgesehen sind, können für eine schwere Qualität mit etwas erhöhter Polsterwirkung und stärkerer Querdrehnbarkeit pro Maschenreihe zwei Strickfäden mit einem elastischen Element verwendet werden.

Schliesslich ist als weiteres Ausführungsbeispiel ein Polsterschlauch der beschriebenen Art mit einer Breite von ca. 30 cm vorgesehen, der für Rumpfpartien bestimmt ist, insbesondere als Polsterung für Gipskorsettes. Die grosse Querdehnbarkeit erlaubt es auch hier, eine Grösse für verschiedene individuelle Anwendungen vorzusehen.

Die Herstellung des Polsterschlauches gemäss der Erfindung erfolgt auf einer Rundstrickmaschine in gefachter Verarbeitungsart, wobei keine prinzipielle Beschränkung der Länge besteht. Für die einzelnen Anwendungen kann die gewünschte Länge vom Verarbeiter individuell abgetrennt werden.

Die Verwendung des beschriebenen Polsterschlauches ist keineswegs auf dessen Anbringung an Körperteilen beschränkt. Insbesondere ist er auch geignet als Ueberzug für individuell geformte, medizinische Unterlagen, wie etwa Gipsbettan. Dazu wird der Polsterschlauch, vorzugsweise mit seiner Frottée-Seite nach aussen über den betreffenden Gegenstand gezogen und passt sich beim Draufliegen vollständig und spannungsfrei der Unterlage an.

## Patentansprüche

1. Medizinischer Polsterschlauch aus gewirktem bzw. gestricktem Textilstoff, der nahtlos zum Schlauch geschlossen ist, dadurch gekennzeichnet, dass jede der umlaufenden Maschenreihen von mindestens einem elastischen Element und mindestens einem Strickfaden gebildet ist, wobei die Maschen Rechts-Links-Bindung aufweisen und als elastisches Element ein Coregarn mit baumwolleumsponnenem, elastischem Kern vorgesehen ist und wobei der Strickfaden auf mindestens einer Seite des Textilstoffes eine frottéartige Struktur bildet, derart, dass der Schlauch längs- und querdehnbar ist, wobei das elastische Dehnungsverhältnis in Längsrichtung grösser ist als in Querrichtung.

2. Medizinischer Polsterschlauch nach einem der vorangehenden Ansprüche, dadurch gekennzeichnet, dass der elastische Kern des Coregarns, eine Elastomerfaser aus Polyurethan aufweist.

3. Medizinischer Polsterschlauch nach einem der vorangehenden Ansprüche, dadurch gekennzeichnet, dass der Strickfaden ein Baumwollgarn ist.

4. Medizinischer Polsterschlauch nach einem der vorangehenden Ansprüche, dadurch gekennzeichnet, dass in jeder Maschenreihe zwei Strikfäden vorgesehen sind.

5. Medizinischer Polsterschlauch nach einem der vorangehenden Ansprüche, dadurch gekennzeichnet, dass er eine Längsdehnbarkeit von mehr als 100% und eine Querdehnbarkeit von mehr als 200% aufweist.

6. Medizinischer Polsterschlauch nach einem der vorangehenden Ansprüche, für Extremitäten, dadurch gekennzeichnet, dass er eine ungedehnte Breite von ca. 8 cm aufweist.

7. Medizinischer Polsterschlauch nach einem der Ansprüche 1 bis 6, für die Rumpfpartie, dadurch gekennzeichnet, dass er eine ungedehnte Breite von ca. 30 cm aufweist.

8. Verfahren zur Herstellung des Polsterschlauches nach einem fer vorangehenden Ansprüche, dadurch gekennzeichnet, dass es mittels einer Rundstrickmaschine in gefachter Verarbeitungsart gestrickt wird.

**Revendications**

1. Manchon rembourré à usage médical réalisé en un textile de bonneterie ou tricoté, sous une forme tubulaire sans couture, caractérisé en ce que chaque rangée périphérique de mailles est formée d'au moins un élément élastique et d'au moins un fil de tricot, les mailles présentant une liaison envers-endroit, un fil enrobé ayant une âme élastique entourée d'un tissu en coton, et en ce que le fil de tricot présente sur au moins un côté du textile une structure du genre frotté, de manière à ce que le manchon soit élastique en direction longitudinale et transversale, avec un rapport d'allongement élastique plus grand longitudinalement que transversalement.

2. Manchon rembourré à usage médical selon une des revendications précédentes, caractérisé en ce que l'âme élastique du fil enrobé comporte une fibre en polyuréthane.

3. Manchon rembourré à usage médical selon une des revendications précédentes, caractérisé en ce que le fil de tricot est un fil de coton.

4. Manchon rembourré à usage médical selon une des revendications précédentes, caractérisé en ce que deux fils de tricot sont prévus dans chaque rangée de mailles.

5. Manchon rembourré à usage médical selon une des revendications précédentes, caractérisé en ce qu'il support un étirement longitudinal excédant 100% et un étirement transversal excédant 200%.

6. Manchon rembourré à usage médical pour extrémités selon une des revendications précédentes, caractérisé en ce que sa largeur non-étirée est d'environ 8 cm.

7. Manchon rembourré à usage médical pour le torse selon une des revendications 1 à 6, caractérisé en ce que sa largeur non-étirée est d'environ 30 cm.

8. Procédé pour la fabrication du manchon rembourré selon une des revendications précédentes, caractérisé en ce qu'il est tricoté au moyen d'une machine à tricoter circulaire fonctionnant avec des fils assemblés.

**Claims**

1. Medicinal upholstery sleeve made of hosiery or knitted fabric shaped as a seamless tube, characterized in that each peripheral row of stitches is formed of at lesat one elastic element and at least a knitting yarn, the stitches having plain and purl knit connections, a core-yarn with an elastic core around which a cotton sheath is spun being provided as the elastic element, and the knitting yarn forming a terry-cloth-like structure on at least one side of the fabric, such that the sleeve can be stretched lengthwise and transversely, the elastic stretching ratio being larger lengthwise than transversely.

2. Medicinal upholstery sleeve according to one of the preceding Claims, characterized in that the elastic core of the core-yarn comprises an elastomeric thread made of polyurethane.

3. Medicinal upholstery sleeve according to one of the preceding Claims, characterized in that the knitting yarn is a cotton yarn.

4. Medicinal upholstery sleeve according to one of the preceding Claims, characterized in that two knitting yarns are provided in each row of stitches.

5. Medicinal upholstery sleeve according to one of the preceding Claims, characterized in that it can be stretched over 100% lengthwise and over 200% transversely.

6. Medicinal upholstery sleeve according to one of the preceding Claims intended for limbs, characterized in that it has an unstretched width of about 8 cm.

7. Medicinal upholstery sleeve according to one of the Claims 1 to 6 intended for the torso, characterized in that it has an unstretched width of about 30 cm.

8. Method for manufacturing the upholstery sleeve according to one of the preceding Claims, characterized in that it is knitted on a circular knitting machine working after the fashion of sleeved yarn.